# EUROPEAN PATENT APPLICATION

(11) **EP 1 048 951 A1**
(43) Date of publication of application: **02.11.2000**
(21) Application number: 99201303.7
(22) Date of filing: 26.04.1999
(51) Int. Cl.: G01N 33/566, G01N 30/46

(54) **Mass spectrometry-based technologies for continuous flow biossays using known ligands**

(71) Applicant: ScreenTec B.V., 2333 CC Leiden (NL)
(72) Inventor: van der Greef, Jan, 3971 BM Driebergen (NL); Irth, Hubertus, 1078 NP Amsterdam (NL)
(74) Representative: Ottevangers, Sietse Ulbe

(57) **Abstract**

The present invention relates to the on-line coupling of mass spectrometry (MS) to continuous-flow separation techniques. In a further embodiment, this on-line detection method is used as either a scanning or monitoring method. Furthermore, this invention relates to compounds detected by this method and the use of these compounds as a ligand for affinity molecules.

## Description

The present invention relates to the on-line coupling of mass spectrometry (MS) to continuous-flow separation techniques. In a further embodiment, this on-line detection method is used as either a scanning or monitoring method for assays, such as biochemical assays, using a known ligand. Furthermore, this invention relates to compounds detected by this method and the use of these compounds as a ligand for affinity molecules.

It is known from the prior art that biochemical assays are highly sensitive detection techniques which combine the selectivity of biospecific interactions with the sensitive detection of labels used as reporter molecules.

An example of such a technique is described in WO 91/13354. This document describes a flow immunosensor wherein an antibody specific to an affinity molecule is immobilized on a support. The antibody's binding sites are saturated with a labeled form of the affinity molecule. A liquid to be analyzed is contacted with the antibody to allow any affinity molecule present in the liquid to displace the labeled antigen. Finally, the displaced labeled antigen is detected.

However, it is known as well that immunoassays suffer from the problem of cross-reactivity, which means that antibodies react with more than one analyte. This leads to erroneous results. For this reason, immunoassays are frequently combined with a fractionation step, e.g., a separation step using HPLC or another type of liquid chromatography.

In the field to which the present invention relates, there is a demand for on-line coupling of a fractionation step and a biochemical assay detection system. Several approaches have been proposed and described to perform continuous-flow biochemical assays. Most of these immunoassays are in the form of a postcolumn reaction detection system based on a sequential addition type immunoassay.

Cassidy et al., Anal. Chem. 64 (1992), 1973-1977 disclose a kinetically controlled immunoassay based on the sequential addition of antibody, sample and label on a protein A column, performing immunoassays for albumin and transferrin in less than 1 minute.

Nilson et al., J. Chromatography, 597 (1992) 383-389 describe a continuous-flow competitive assay involving enzyme-labelled antibodies. The system described was coupled to a size-exclusion column to allow the monitoring of activity.

These prior art techniques which are of a sequential nature are not suitable for on-line coupling to liquid chromatographic or other fractionation systems, since these techniques do not allow the continuous monitoring of the fractionation effluent.

In the article of Irth et al. in the Journal of Chromatography 633 (1993) 65-72 and in the articles of Oosterkamp et al. in Anal. Chem. 66 (1994) 4295-4301, and in the Journal of Chromatography 653 (1994) 55-61, a method for the on-line detection of digoxigenin and its metabolites is described. The on-line detection process comprises the direct injection of a sample containing digoxigenin and its metabolites, a liquid chromatographic (LC) fractional separation step, the mixing of the effluent of the LC column with fluorescein-labelled antibodies against digoxigenin, the removal of free labelled antibodies from the mixture via passage through a small column packed with an antigen-bound support, and detection of the strongly fluorescent immunocomplexes.

The digoxigenin system described is based on association reactions of antibodies and antigens eluting from the analytical column. By the use of fluorescein-labelled antibodies detection limits in the nanomolar range are obtained.

This prior art assay is a heterogeneous detection system. It requires a separation step between free and bound label. Examples for free/bound label separation techniques are restricted-access phases and hollow-fibre modules.

The immunoreagent in the previously described prior art assay consists of fluorescein-labelled fragments of anti-digoxigenin antibodies which were immunopurified and are commercially available. The commercial availability of purified, labelled antibodies is however exceptional. In almost all cases, antibodies are only available in unlabelled state in crudely purified antiserum. Although labelling and purification schemes for antibodies (or other affinity proteins) are known to the person skilled in the art, it will be preferred to use antisera, which may be commercially available, without any pretreatment.

Furthermore, it is difficult to selectively label only those antibodies in an antibody preparation which react with the analytes. In general, labeling techniques make use of primary amine groups of antibodies resulting in the co-labeling of all other proteins present in the preparation. This leads to a drastic increase in the background signal and, consequently, to an increase in detection limits.

One aim of the invention is to provide alternatives for this known technique, viz. providing a technique which does not require the use of labeled antibodies. In addition, another aim of the present invention is to provide a technique that is not dependent on fluorescent behavior of labels, which makes it more versatile.

Hsieh et al. (Molecular Diversity, 2 (1996), 189-196) describe a screening method using chromatography coupled with mass spectrometry. Their method requires a dual run, first in the absence of a library of protein targets, and then with the library. This means that background compounds, especially the affinity proteins causing severe background signals over a broad range in the mass spectrum, are always present in this assay type, which limits the efficiency, selectivity and flexibility of the method considerably.

Kaur et al. (Journal of Protein Chemistry 16, No 5 (1997)) describe a method in which active components in a combinatorial library are bound to a receptor, isolated by size exclusion chromatography and trapped on a column after which the desorption solvent is used to dissociate the affinity protein/ligand complex. Subsequent identification is done by mass spectrometry, but with a background of the affinity protein.

The methodology described by Hsieh and Kaur is thus hampered by severe background signals in the mass spectrometer of the present affinity protein.

The invention seeks to overcome the above mentioned problems. This is achieved by the provision of an on-line detection method comprising the on-line coupling of the effluent a fractionation step and a mass spectrometer (MS), which method comprises the addition of a controlled amount of an affinity molecule, such as an affinity protein or a receptor, to an effluent of the fractionation step, whereby the affinity molecules bind analytes in the effluent, followed by the addition of a controlled amount of a known ligand capable of binding to the affinity molecule under suitable binding conditions, followed by a separation step to separate the free and bound known ligands and finally detection of either the free or bound known ligands using the mass spectrometer.

The change in the amount of known ligand as detected by the MS indicates the presence of an affinity molecule-binding compound in the effluent.

In particular, in a first step, a suitable affinity molecule for analytes to be detected - which affinity molecule may e.g. be an affinity protein, such as an antibody or avidin - is added to the effluent of a liquid chromatography or capillary electrophoresis column to react with an analyte eluting from the column. Unbound affinity molecules react in a second step with an excess of a known ligand to titrate the remaining free binding sites; or as an alternative, a competition reaction occurs between the affinity molecule, the analyte in the effluent and the known ligand. Normally, the ligand/affinity molecule complex is detected after a separation of free and bound ligand, preferably on the basis of the difference in molecular weight. Finally, the ligand/affinity molecule complex is detected, either directly or after a dissociation step, with the MS.

The combination of the fractionation step with the MS detection step in accordance with the present invention greatly enhances the performance of both techniques. The combined techniques provide an analytical method which is characterized by a high selectivity and a high sensitivity. Further, the problem associated with cross-reactivity does not occur when using the method of the present invention. Furthermore, the assays with the method according to the present invention can be performed quantitatively.

The method of the present invention uses bioaffinity molecules such as antibodies, receptors etc. to detect any compounds showing high affinity for the ligand binding site of said affinity molecule. The compounds to be detected may be biochemical compounds but are not in any way restricted thereto.

The method of the invention makes it possible to screen a mixture of different compounds on their ability to bind to a certain known ligand, e.g., in order to find an inhibitor for said ligand. Therefore, the present invention further relates to a fast on-line method for the screening of compounds for their binding capability to a known ligand, which method comprises a fractionation step providing an effluent, the addition of a controlled amount of said affinity molecule to the effluent of the fractionation step and effecting a contact time sufficient to allow a reaction with or an interaction between the compounds in the effluent, the subsequent addition of a controlled amount of a known ligand capable of binding to the affinity molecule, and either direct detection of the amount of free ligand or the indirect detection of ligand/affinity molecule complex by first dissociating the ligand from the complex and subsequent detection of the dissociated ligand, both using the MS. A change in the amount of detected affinity molecule/detectable ligand complex indicates the presence of an affinity molecule-binding compound in the effluent.

A comparison of the MS data obtained when analytes are injected with the signal obtained when only the controlled amounts of affinity molecule and detectable ligand are introduced in the continuous-flow system, provides informat.ion in respect of the percentage of the binding sites occupied by analytes present in the effluent of the fractionation step. The person skilled in the art possesses the knowledge to process and evaluate the data obtained from the detection method. The percentage binding sites occupied by an analyte present in the effluent can be used to find new compounds which show an interaction with a known affinity molecule. This information provides the possibility to implement the on-line separation/affinity molecule detection process of the present invention in, e.g., drug discovery.

A system of high-throughput screening to be used in drug discovery, for instance, consists of the following steps. Complex samples generated for instance by an upstream combinational chemistry system are prefractionated in fractions containing compounds of similar polarity using, e.g., a solid-phase extraction technique or electrophoretic sample handling principles. Each fraction may additionally be separated using, e.g., either analytical or preparative-scale liquid chromatographic separation columns. The compounds eluting from said LC column are on-line detected using a suitable affinity molecule detection technique. Where preparative-scale separation columns are applied, a postcolumn flow-split will be made. One of the two flow streams is subjected to detection using the affinity molecule detection technique; the other stream is directed to a fraction collector. Dependent on the signal obtained from the affinity molecule detector, fractions containing compounds causing a positive response will be collected while fractions causing a negative response will be discarded. This complete screening method can be automated using known valve-switching processes.

A suitable fractionation method to be used in the methods of the present invention comprises a liquid chromatography separation or a capillary electrophoresis step. Other separation or fractionation techniques which are known to the person skilled in the art and which allow a relatively continuous output stream can, however, be used as well.

In a preferred embodiment, the liquid chromatography separation step is a reversed phase HPLC step.

It will be understood that according to the present invention as the effluent of a fractionation step is also to be understood the effluent of a flow injection, in which a mixture of different compounds is injected, optionally followed by a trapping step, for example using a restricted-access column. The separation step of the method according to the present invention then fulfills the fractionation, since the compounds of interest are fractionated by the separation step. The required selectivity can be obtained by operating the MS selectively tracing of the MS.

Since the flow injection technique provides a flexible and fast screening method, it is a preferred embodiment of the present invention.

All modes of MS-operation are possible in Flow Injection mode, however, due to the higher background, especially the very selective modes which comprise detecting ions of a selected single m/z trace or of selected multiple m/z traces are suitable.

In a preferred embodiment, the separation step comprises the retention of the free ligand from the effluent using a restricted-access support, whereby the ligand-affinity molecule complex is permeated, and the bound ligands are detected after being separated from said ligand-affinity molecule complex in a suitable dissociation step, followed by separation of the ligand from the affinity molecule using a hollow-fiber module, and directing the permeate stream containing the ligand to the mass spectrometer, in which method the dissociation step is preferably a low pH shock, contacting with a high ionic strength solution, contacting with an organic solvent and/or contacting with a chaotropic reagent.

Restricted-access supports are well-known to the person skilled in the art. In general, restricted-access supports, such as C₁₈-silica packed columns, are capable of retaining smaller sized molecules while permeating the larger molecules.

Hollow fiber materials, for example made of polysulphon, are well-known to the person skilled in the art. In general, hollow fiber modules are capable of permeating smaller sized molecules while retaining the larger molecules.

In another preferred embodiment, the separation step comprises the retention of the ligand-affinity molecule complex from the effluent using a hollow-fiber module, whereby the free ligand is permeated, and the permeate stream with the free ligand is subsequently directed to the mass spectrometer.

In yet another preferred embodiment the separation step comprises the retention of the free ligand from the effluent using a restricted-access support, whereby the ligand-affinity molecule complex is permeated, followed by elution of the unbounded ligands from the restricted-access support using a suitable carrier stream, and directing the eluted stream containing the free ligand to the mass spectrometer.

As already mentioned, the fractionation step can be a liquid chromatography separation, a capillary electrophoresis step or a combinatorial chemistry system. It has been found that it is vary advantageous when this fractionation step is followed by a separation step, for example by using a hollow-fiber module, since this removes the high molecular weight background. Preferred liquid chromatography fractionation steps comprise HPLC, reversed phase HPLC, capillary electrophoresis (CE), capillary electrochromatography (CEC), isoelectric focusing (IEF) or micellar electrokinetic chromatography (MEKC), all of which techniques are known to the person skilled in the art.

All possible variations in MS techniques known in the art can be profited from according to the present invention. preferably, the MS is of the type chosen from the group consisting of electrospray ionization type, atmospheric pressure ionization type, quadrupole type, magnetic sector type, time-off-flight type, MS/MS, MSⁿ, FTMS type, ion trap type and combinations thereof.

For example, in scanning mode to trace compounds, low resolution MS with all possible instrumental designs of MS can be used, in particular quadrupole, magnetic sector, time-off-flight, FTMS and ion-trap. This generates typically molecular weight data with nominal mass accuracy.

When high resolution MS is applied, using all possible high resolution instrumental designs, in particular magnetic sector, time-off-flight, FTMS and ion trap, molecular weight data with high mass accuracy combined with the elemental composition of the compound can be obtained.

Other known MS techniques comprise tandem MS, such as MS/MS or MSⁿ (for example MS³). Application of these techniques enables the collection of structural information of the ligands which is a preferred embodiment of the present invention. The data in scanning mode can be acquired in data-dependent mode which means that for each peak observed automatically the tandem MS measurement is performed. In addition to this the fragmentation induced in the tandem MS measurement can be coupled by more sophisticated procedures, for example a broad band excitation, which also fragments expected fragments of less importance, such as the [protonated molecule-H₂O]⁺ peak in natural product screening.

The MS can also be operated in single/multiple ion monitoring mode, which can be used for highly selective detection.

Single/multiple ion monitoring mode can be used for highly selective detection. Using low resolution MS in single/multiple ion monitoring mode with all possible low resolution instrumental designs, in particular quadrupole, magnetic sector, time-off-flight, FTMS and ion trap, selective detection based on monitoring of previously determined m/z trace can be obtained. When high resolution MS is applied all possible instrumental designs of MS can be used, especially quadrupole, magnetic sensor, time-off-flight, FTMS and ion-trap. This enables typically very selective detection based on monitoring in high resolution previously determined m/z trace. When tandem MS is applied, all possible instrumental designs, in particular ion trap, quadrupole-time-off-flight (Q-TOF), triple quadrupoles (QQQ), FTMS and combinations of sector instruments with quadrupoles and ion traps, can be used. This enables the very selective detection based on monitoring a resulting peak or peaks in MS/MS and/or MSⁿ measurements.

The known ligand assays according to the present invention are based on continuous monitoring of the HPLC effluent after a separation step, for instance with a hollow fiber step, to remove the high molecular weight background. The MS is typically set to operate in one of single/multiple ions mode options. The activity is either detected by an increase (direct detection) or a decrease (indirect detection) of the pre-selected monitoring signals, based on the MS-characteristics of the known ligand.

It is also possible to operate the MS in scanning mode. In this way the lowering of the pre-determined signal is correlated to the increase of other signals, enabling the active compound to be characterized in the same cycle.

With the assays according to the present invention the tracing of (bio-) active compounds in solutions of complex nature, for instance biological fluids or extracts, natural product extracts, solutions or extracts from biotechnological processes, resulting from chemical experiments, such as combinatorial technologies and processes and the like can be performed with a higher efficiency, selectivity and flexibility. Moreover, the present invention provides the possibility to limit the disturbance of background compounds.

The present invention further enables the identification of compounds based on conventional mass spectra, high resolution data or MSⁿ based spectra.

With the method of the present invention it is also possible to perform library searching, based on a variety of mass spectrometric experiments enabling the screening of large series of samples and classifying these in classes based on similar active compounds, without the need for full identification of the compounds.

The assay method of the present invention can be applied in miniaturized formats of assay-based systems, for instance with chip technology based screening systems.

In the methods of the present invention all molecules capable of interaction with or binding to other molecules can be used as affinity molecule. The affinity molecule can for example be selected from the group of cytosolic receptors, e.g., the estrogen, glucocorticoid receptors; solubilized membrane bound receptor, e.g., a β-receptor; affinity proteins, such as antibodies, enzymes, avidin; polynucleotides and polysaccharides.

By a controlled amount of affinity molecule that is added to the effluent is to be understood an amount of known concentration and of known flow rate.

The term "known ligand" as used in the present description and claims refers to a ligand which is capable of interacting or reacting with the above-defined affinity molecule, and which can be detected by the MS. An example of a detectable ligand is a previously found analyte capable of being bound by the affinity molecule.

The present invention has the great advantage that the preparation of any label is not required. This is particularly advantageous for those affinity molecules where labeling of a known ligand leads to a dramatic loss of affinity, for example due to steric hindering. Assays using known, natural, ligands without labels as reporter molecules are much faster to develop.

On-line coupling as used in the methods of the present invention requires fast reaction times in order to minimize extra-column band broadening. This means that affinity molecule-ligand interactions having reaction times in the order of minutes rather than hours should be considered. The suitable binding conditions under which the affinity molecules bind to the analyte comprise a contact time, which is in the same order of magnitude. Suitable binding conditions are conditions that provide optimal binding between the affinity molecules and the analyte. It will be understood that the precise conditions, such as temperature, residence time, chemical composition, will depend strongly on the type of assay and the proteins used therein.

## Claims

1. On-line detection method comprising the on-line coupling of the effluent of a fractionation step to a mass spectrometer, which method comprises the addition of a controlled amount of an affinity molecule to an effluent of the fractionation step, whereby the affinity molecules bind analytes in the effluent, followed by the addition of a controlled amount of a known ligand capable of binding to the affinity molecule under suitable binding conditions, followed by a separation step to separate the free and bound known ligands and finally detection of either the free or bound known ligands using the mass spectrometer.

2. On-line detection method according to claim 1, in which the separation step comprises the retention of the free ligand from the effluent using a restricted-access support, whereby the ligand-affinity molecule complex is permeated, and the bound ligands are detected after being separated from said ligand-affinity molecule complex in a suitable dissociation step, followed by separation of the ligand from the affinity molecule using a hollow-fiber module, and directing the permeate stream containing the ligand to the mass spectrometer, in which method the dissociation step is preferably a low pH shock, contacting with a high ionic strength solution, contacting with an organic solvent and/or contacting with a chaotropic reagent.

3. On-line detection method according to claim 1, in which the separation step comprises the retention of the ligand-affinity molecule complex from the effluent using a hollow-fiber module, whereby the free ligand is permeated, and the permeate stream with the free ligand is subsequently directed to the mass spectrometer.

4. On-line detection method according to claim 1, in which the separation step comprises the retention of the free ligand from the effluent using a restricted-access support, whereby the ligand-affinity molecule complex is permeated, followed by elution of the unbounded ligands from the restricted-access support using a suitable carrier stream, and directing the eluted stream containing the free ligand to the mass spectrometer.

5. Method according to any of the preceding claims in which comprises a fractionation step which is a liquid chromatography separation, a capillary electrophoresis step or a combinatorial chemistry system, which is optionally followed by a separation step which removes the high molecular weight background.

6. Method according to claim 5, in which the liquid chromatography separation step is a HPLC, a reversed phase HPLC, a CE, a CEC, a IEF or a MEKC step.

7. Method according to any one of the preceding claims, in which the mass spectrometer is of the type chosen from the group consisting of electrospray ionization type, atmospheric pressure ionization type, quadrupole type, magnetic sector type, time-off-flight type, MS/MS, MSⁿ, FTMS type, ion trap type and combinations thereof.

8. Method according to any one of the preceding claims, in which the mass spectrometer is set to detect ions of a selected single m/z trace or of selected multiple m/z traces.

9. Compound detected by the method of any one of the preceding claims.

10. The use of a compound of the preceding claim as a ligand for affinity molecules.
